# EUROPEAN PATENT APPLICATION

(11) **EP 1 317 927 A1**
(43) Date of publication of application: **11.06.2003**
(21) Application number: 01204704.9
(22) Date of filing: 05.12.2001
(51) Int. Cl.: A61K 35/14, G01N 33/50, C12N 5/08, A61K 39/00, A61P 35/00

(54) **Minor histocompatibility antigen HA-1: target antigen for immunotherapy of tumors**

(71) Applicant: Academisch Ziekenhuis Leiden, 2333 ZA Leiden (NL)
(72) Inventor: Goulmy, Elsa Afra Julia Maria, 2343 BC Oegstgeest (NL)
(74) Representative: Prins, Adrianus Willem

(57) **Abstract**

Allogeneic stem cell transplants (SCT) can induce curative Graft versus Tumor (GvT) reactivities in patients with hematological malignancies. The GvT reaction is mainly mediated by allo immune donor T-cells specific for polymorphic minor Histocompatibility antigens (mHags). Among these, the mHag HA-1 was found to be restricted to the hematopoietic system. Here we report the expression of HA-1 by non-hematopoietic tumor cells. While absent in normal epithelial cells, tumor cells, tumor cell lines particularly from epithelial origin, also express HA-1 and are recognized by HA-1 cytotoxic T cells. The invention provides, among others, means and methods for HA-1 specific immunotherapy for HA-1 positive patients with non-hematopoietictumor cells.

## Description

The invention relates to the field of immunology, particular to the field of immunotherapy and prophylaxis of cancer.

Clinical and experimental data indicate that allogeneic Stem Cell Transplantation (SCT) not only reconstitutes the patient's hematopoietic system but mediates a powerful curative effect in patients transplanted for hematological malignancies or for solid tumors (1-7). These allo immune Graft versus Host (GvH) reactivities generally lack tumor specificity and are often accompanied by severe Graft versus Host Disease (GvHD). Target antigens for successful immunotherapy of cancers therefore preferably have: tissue specificity, functional membrane expression on the tumor cells and the capacity of inducing (allo) immune T cell responses. The GvH reactivities after HLA identical SCT are attributed to antigens encoded by genes other than the Major Histocompatibility Complex (MHC). These other antigens are generally referred to as minor Histocompatibility antigens (mHags) (8). mHags are peptides from polymorphic intracellular proteins that are encoded by genes on the Y-chromosome and by autosomal genes independent from HLA. Their immunogenicity arises as a result of their expression on the plasma membrane where they are recognized by alloreactive MHC restricted T cells (9). We demonstrated earlier that mHags show either ubiquitous or restricted tissue expression (10). The tissue expression of the mHag HA-1 is limited to the hematopoietic cells only. Functional studies with HA-1 specific cytotoxic T lymphocytes (CTLs) demonstrated efficient lysis of hematopoietic cells, including leukemic cells (10, 11) and inhibition of leukemic progenitor cell outgrowth (12), whereas no CTL recognition was observed when non-hematopoietic cells were used as target cells (10).

In the present invention, HA-1 was detected in tumor cells that were not of hematopoietic origin. HA-1 RNA transcription was demonstrated in cell lines that were generated from a wide array of tumors i.e. breast, melanomas, lung, renal cell and colon carcinomas, hepatomas and head and neck cancers. Of these cell lines, the cell lines expressing HLA-A2 and HA-1 phenotypes, were lysed by HLA-A2 restricted HA-1 specific CTL. This lysis demonstrates that HA-1 is indeed expressed in a functional way in the tested cells. That also primary cancer cells express HA-1 was verified by RNA analysis. Also disseminated cancer cells express HA-1. Disseminated cells from six of fifteen patients were positive for HA-1. Expression of HA-1 is not limited to a certain type of tumor cell. HA-1 expression was found on different types of carcinoma cells in the patient population.
The observation that HA-1 is expressed in a functional way on the membrane of tumor cells of non-hematopoietic origin opens the road to many different applications. One application is the use of HA-1 expression on the tumor cell to target therapy to that cell. In one aspect the invention therefore provides a method for eliminating a tumor cell presenting a HA-1 minor Histocompatibility antigen in the context of HLA class I, wherein said elimination is induced directly or indirectly by specific recognition of said mHag in said context, the method characterized in that said aberrant cell comprises a non-hematopoietic tumor cell that expresses HA-1. Preferably, said tumor is an epithelial tumor cell.

There are several ways to induce elimination of a cell through specific recognition of a target on that cell. In the present invention emphasis is put on specific recognition by T-cells, however, the invention is not limited to T-cells. Targeting is also possible with molecules. Any type of molecule capable of specifically recognizing said mHag in said context is suitable, provided that the molecule can mediate elimination of the cell, either directly or indirectly (by means of a toxic effect) or indirectly, through binding of another molecule that comprises a toxic effect. In one embodiment, said elimination is achieved through specific recognition by a murine or human(ized) antibody specific HA-1 or specific for HA-1 presented in the context of MHC. Humanized or human monoclonal antibodies (though with different specificities) are used in, or developed for, a great variety of anti-tumor therapies in the clinic.

A preferred means for inducing elimination of HA-1 expressing tumor cells comprises elimination induced by a T cell comprising a T cell receptor specific for HA-1 presented in the context of MHC class-I. This technology ties in with strategies for adoptive immunotherapy for hematopoietic malignancies. Malignant cells derived from the hematopoietic system can express HA-1 and can therefore form the target for T-cells comprising a specificity for HA-1 presented in the context of MHC class-I. With the teaching of the invention it is possible to extend these approaches to any type of tumor cell of non-hematopoietic origin. The adoptive immunotherapy methods for hematopoietic malignancies are therefore also part of the invention and are incorporated herein. The cell directly involved in killing in these adoptive immunotherapy approaches is a cytotoxic T-cell. The invention thus also provides a method for killing a human cell functionally expressing a HA-1 mHag in the context of HLA class I, comprising incubating said cell with a cytotoxic T lymphocyte (CTL) specific for said mHag presented in said context or incubating said cell with a functional equivalent of said CTL, the method characterized in that said human cell comprises a non-hematopoietic tumor cell. The invention further provides a method for determining whether a cell expresses functional levels of an HA-1 mHag in the context of HLA class I, comprising incubating said cell with a cytotoxic T lymphocyte (CTL) specific for said HA-1 mHag presented in said context and determining whether said cell and/or said CTL is affected. There are several way to determine whether said cell or said CTL is specifically affected by the incubation. One typically uses target cell killing to determine specific recognition by CTL, however, detection of gene expression characteristic for CTL mediated lysis in the CTL or target cell can also be used.

Now that the invention demonstrates that HA-1 is expressed in non-hematopoietic tumor cells, said cells can be detected and discriminated from normal cells using methods for specifically detecting HA-1 in a cell. Typically though not necessarily said detection methods utilize binding molecules capable of binding specifically to HA-1 and/or nucleic acid encoding said HA-1. For detection it is not required that HA-1 is presented in the context of MHC-I. Indeed preferably, said HA-1 or encoding nucleic acid is present in the context of the normal protein/gene. The invention therefore further provides a method for marking a non-hematopoietic tumor cell comprising incubating said cell with a molecule capable of specifically binding to an HA-1 mHag presented in the context of HLA class I, or capable of specifically binding to a nucleic acid encoding said HA-1 mHag. In principle any type of method for specifically determining the presence of a particular expression product is suitable for the present invention and is provided herewith. Of course, also provided is a non hematopoietic tumor cell comprising a molecule capable of specifically binding to an HA-1 mHag presented in the context of HLA class I, or capable of specifically binding to a nucleic acid encoding said HA-1 mHag.

Methods of the invention can be performed in vitro, however, in a preferred embodiment a method is performed in vivo. In vivo, a method of the invention can be used for the prevention and/or treatment of diseases caused by tumor cells. Provided is a method for at least in part inhibiting expansion of a tumor in an individual comprising providing said individual with a CTL specific for an HA-1 mHag presented in the context of HLA class I, or a functional equivalent of said CTL, the method characterized in that said tumor cell comprises a non-hematopoietic tumor cell presenting said HA-1 mHag in the context of said HLA class I. To obtain inhibition of expansion or even a reduction in tumor mass it is not required that all of said tumor cells express the HA-1. Though non-HA-1 expressing cells are not eliminated the removal of HA-1 expressing cells can still be relevant for treatment. Thus inhibition of expansion of tumor cells can also be achieved when only a part of the tumor cells express HA-1. The method may be performed in combination with other means of tumor cell removal. In a preferred embodiment the method is used to combat recurrence of tumor in situations of minimal residual disease. Of relevance for in vivo applications is the fact that normal hematopoietic cells also express HA-1. Thus any method capable of specifically eliminating cells presenting HA-1 in the context of MHC class I, will in vivo also affect a hematopoietic system. To this end it is preferred to provide the individual with hematopoietic cells that are resistant to lysis by said CTL. This can be achieved in several ways. Thus, the individual is transplanted with hematopoietic stem cells comprising a different or no HA-1 and/or different MHC class-I alleles. In a preferred embodiment said individual is transplanted with hemopoietic cells from a HA-1 negative donor. These stem cells can not be recognized by the CTL and can thus not be lysed by the T cell. In a preferred embodiment said stem cell comprises is negative for HA-1 or comprises a different HA-1 but the same MHC class-I compared to said tumor cell. A method for elimination or killing of a tumor cell of the invention is particularly suited for the treatment of metastases. Preferably in the form of minimal residual disease, in particular liver metastases.

Cells for transplantation may be obtained directly from a donor. However, current culture technology allows significant expansion of cell in vitro, without detrimental effects on fitness and integrity of the cultured cells. Thus cells for transplantation and T-cells can be cultured in vitro if need be. A possibility is to use T cells that are educated ex vivo to comprise T cell receptors capable of binding to HA-1 presented in the context of MHC class I. These educated T-cells can be expanded further ex vivo before providing them to an individual. A fully ex vivo approach toward education and expansion of the right T cells has the advantage that the cells can be analyzed and safety tested extensively before transplantation A.CTL of the invention can also be generated by incubating T cells with dendritic cells comprising said MHC class-I and said HA-1. The dendritic cells may be provided with said HA-1 by contacting with a peptide comprising said HA-1. Such systems also allow the generation of banks with extensively characterized and tested T cells with known specificities. Current and future technologies may be used to generate the HA-1 specific T-cell of the invention. For instance, current methods for the generation of such T-cell include introduction of the genetic information for the relevant T-cell receptor into cells that are already T cells or that can become T-cells. On the other hand functional equivalents of T cells of the invention are also foreseeable. For instance, a suitable cloned T-cell receptor may be introduced in so-called empty cells that are T-cells from which the relevant T-cell receptor is may dysfunctional. Such T-cells do not express functional levels of native T-cell receptor chains and can thus not provide for chimearic T-cell receptors with unexpected specificities when provided with a HA-1 specific T cell receptor.

In one embodiment T-cell and/or other hematopoietic cells provided to said individual comprise additional features. Such additional features can for instance comprise safety features, or additional (co)-stimulation features. Safety can be built in for instance using so-called suicide genes like Herpes Simplex Virus Thymidine Kinase (HSV-TK). Expression of HSV-TK is toxic for many cells when a pro-drug like gancyclovir is provided to the cells. A safety feature can be built in for a variety of reasons, one of which is a relatively simple way to down-regulate the number of grafted cells in the body in case of undesired effects of the grafted cells (GvhD and/or neoplasia).

Other features can be built in for instance features to improve the anti-tumor effect of the grafted T-cells. This can be done for instance by introducing into the cells co-stimulatory factors, cytokines and/or the encoding genes therefore. Thus in a preferred embodiment a method is provided wherein said individual is provided with said CTL by providing said individual with a graft comprising hematopoietic cells of a donor.

In methods of the invention T cells comprising a T cell receptor specific for HA-1 presented in the context of MHC class-I are being generated, isolated, manipulated and provided with additional features. T-cells obtained with a method of the invention are therefore also part of the invention. With the current pace of development in biological methods and knowledge it is foreseen that cells can be made into antigen specific T cells in an artificial way, for instance through manipulating programming genes. When such cells are made specific for HA-1 presented in the context of MHC class I than such cells are equivalent to T-cells of the invention. T cells of the invention and equivalents thereof can be the basis for the preparation of medicaments for tumor cells. The invention thus also provides the use of an antigen specific T cell or an equivalent thereof comprising a specificity for HA-1 presented in the context of MHC class-I for the preparation of a medicament for the treatment of disease related at least in part to non-hematopoietic tumor cells. Provided is also a use of a molecule capable of specifically binding an HA-1 mHag in the context of HLA class I for preparing a medicament for the treatment of disease related at least in part to non-hematopoietic tumor cells.

Using a method of the invention it is possible to at least in part inhibit the growth of a non-hematopoietic tumor in an individual. In an allogeneic SCT setting, donor lymphocyte infusion (DLI) therapy has been clearly shown curative for hematological malignancies (20, 21). However DLI therapy is associated with GvHD. To treat leukemia relapse after HLA matched, mHag HA-1 mismatched SCT with low risk of GvHD, we have previously developed ex vivo protocols for the generation of donor derived CTLs specific for the hematopoietic specific mHag HA-1 (22). These SCT donor derived HA-1 specific CTLs eliminate the HA-1 positive patient' hematopoietic and leukemic cells, while HA-1 negative non-hematopoietic cells and tissues will be spared. In the HLA identical allogeneic SCT setting for solid tumors, GvT reactivity has been demonstrated in small cohorts of patients with metastatic cancers, including breast cancer (4, 5, 23), melanomas (6), renal cell carcinomas (3) and ovarian carcinoma (7). From the present invention we know that at least part of this GvT reactivity is due to tumor specific polymorphic mHags such as HA-1. As in the leukemia transplant patients where residual leukemic tumor cells are present after high-dose chemotherapy, HA-1 directed immunotherapy is particularly warranted in cancer patients with minimal residual tumor cells who were shown to confer an increased risk for a later occurring relapse (24). Our observation of HA-1 expression on various types of non-hematological tumor cells, offers a novel target molecule for therapy. Similar to the cellular immunotherapy protocol for the treatment of relapsed leukemia, as described above, adoptive immunotherapy with donor derived HA-1 CTLs in combination with SCT is an attractive alternative treatment of non-hematopoietic tumors, preferably, solid tumors. Because of the hematopoietic expression of the HA-1 gene, we expect the patient's hematopoiesis will be eliminated and needs reconstitution from donor SC or equivalents thereof. Based on HA-1 restricted expression on malignant non-hematopoietic tissues, HA-1 cellular therapy should be specific, with no foreseen damage to normal tissues and cells. The above mentioned HA-1 based immunotherapy capitalizes on expression of the ligand for HA-1 CTL recognition, which is HLA-A2 and the HA-1^{H} allele of the HA-1 locus. Since there are many different HLA-molecules it is expected that at least some other HLA molecules are also capable of presenting HA-1. Methods of the invention are also suitable for these other HLA molecules. The HA-1^{H} phenotype frequency is 69% in the HLA-A2 positive population (25). To our knowledge this is the first example of a constitutive hematopoietic specific gene that can function as a universal tumor specific antigen, with no significant expression on its non-malignant counterparts. The significance of the polymorphic mHag HA-1 for cancer therapy is underscored by the known HA-1 immunogenic functional membrane expression and adequate CTL recognition.

It may become relevant to counteract an immune response against HA-1 in an individual. This may for instance be the case when the immune response is no longer needed or when rescue of autologous cell is desired, for instance in case of inadvertent negative effects of foreign HA-1 specific T-cells. An infused cell can for instance become neoplastic, or otherwise deregulated in its cytokine excretion or responsiveness. Thus an efficient way of counteracting an immune response against HA-1 is usefull for a variety of reasons. Some way's to counteract are given elsewhere in this application. Here another way of counteracting is provided. The present invention in one aspect provides a method to modulate mHag HA-1 response by providing an antagonistic peptide. A peptide can be provided to an MHC-I expressing cell in many ways, for instance through providing the peptide, or the encoding nucleic acid. The peptide can be designed such that it comprises a sufficiently strong affinity for the HLA-molecule that HA-1 peptide is effectively prevented from associating with HLA, thereby at least in part reducing the capability of the HA-1 specific immune response to attack said cell.

In yet another aspect the invention provides a method for the treatment of an individual suffering from or at risk of suffering from a non-hematopoietic tumor comprising inducing and/or enhancing in said individual an immune response against HA-1 presented in the context of HLA class I. In one embodiment said immune response is induced and/or enhanced by administering a CTL specific for HA-1 presented in the correct context. In another embodiment said immune response is induced and/or enhanced by vaccinating said individual with a (poly)peptide comprising HA-1 antigen. Vaccinations may be performed using any method for vaccination against a peptide known in the art. A preferred means of vacination comprises the so-called string of beads method of vaccination wherein several different peptides are incorporated into a proteinaceous molecule. When HA-1 antigen is provided in the context of a larger molecule it is preferred that the peptide comprising the HA-1 antigen is flanked at least on one side but preferably on both sides by appropriate processing sites to allow cutting of the HA-1 antigen and the transport said antigen to the relevant site and the association of the antigen with the appropriate MHC-I molecule. Vaccinations can of course also be performed using other methods known in the art. Such methods preferably comprise MHC tetramers. Vaccinations may be performed in the traditional sence or vaccination may be performed using artificial antigen presenting moieties in the form of liposomes comprising such MHC presenting the relevant HA-1 antigen. Vaccinations may of course comprise any suitable type of adjuvant. Preferably, the adjuvant comprises CpG rich genes. This adjuvant is particularly preferred when vaccination is performed with nucleic acid coding for an expressible HA-1 antigen.

In general peptides presented in the context of HLA vary in length from about 7 to about 15 amino acid residues, and a polypeptide can be enzymatically processed to a peptide of such length. A peptide comprising HA-1 antigen provided by the invention typically is at least 7 amino acids in length but preferably at least 8 or 9 amino acids. The upper length of a peptide provided by the invention is no more than 15 amino acids, but preferably no more than about 13 or 11 amino acids in length. A peptide provided by the invention contains the necessary anchoring residues for presentation in the groove of the relevant HLA molecule. An immunogenic polypeptide provided by the invention comprises a 7-15 amino acid long peptide, optionally flanked by appropriate enzymatic cleavage sites allowing processing of the polypeptide. A preferred embodiment of the present invention is a peptide with the sequence VLHDDLLEA which induces lysis of the cell presenting it at a very low concentration of peptide present. This does not imply that peptides inducing lysis at higher concentrations are not suitable. This will for a large part depend on the application and on other properties of the peptides, which were not all testable within the scope of the present invention. Presentation of the HA-1 antigen by MHC-I can occur in various ways depending on the particular type of MHC-I. Different HLA molecules behave differently in their capacity to present a peptide. In the present invention HA-1^{H} antigen can be presented by different HLA molecules. In case of HLA-A2 the peptide presented comprises the sequence VLHDDLLEA. When the HLA molecule is HLA-B60 the HA-1^{H} antigen comprises a sequence that is shifted slightly when compared to the sequence presented by HLA-A2. However, the polymorphism is of course still present in the peptide presented by HLA-B60. Thus the HA-1 antigen may comprise any peptide capable of being presented by an MHC-I or for that matter MHC-II molecule provided that it comprises the relevant polymorphism.

The peptides and other molecules according to the invention find their utility in that they induce and/or enhance the immune induced elimination of non-hematopoietic tumor cells. Since the hematopoietic cells of the HA-1 positive recipient also express HA-1, it is preferred that the individual wherein an immune response against HA-1 in the context of HLA, is induced and/or enhanced is provided with HA-1 negative hemopoietic stem cells. The mentioned HA-1 antigen containing (poly)peptides can be used to prepare therapeutic agents capable of eliminating a subset of cells, directly or indirectly, especially tumor cells of non-hematopoietic origin. This can be illustrated by the following examples, which refer to leukemia related therapeutic agents.
A HA-1 positive, non-hematopoietic tumor baring recipient (in bone marrow transplantation) can be subjected to an additional pre-bone marrow transplant conditioning regime. This means that an agent which specifically recognizes a (poly)peptide according to the invention (a HA-1 comprising (poly)peptide) as presented selectively on hematopoietic cells, which agent induces elimination of the cells presenting said peptide, is administered to the recipient before transplantation. This agent will eliminate all (residual) tumor cells and cells of hematopoietic origin. Such agents include but are not limited to T cells (which are tailor made ex vivo by pulsing with the peptides provided by the invention, and optionally provided with a suicide gene) and/or antibodies coupled to toxic moieties. A HA-1 negative donor for bone marrow transplantation can be vaccinated with a peptide according to the invention, a HA-1 peptide. Upon transplantation to a HA-1 positive recipient, the donor's immune system can eliminate any residual or recurrent HA-1 peptide presenting cells in the recipient which are of course leukemic. This is another example of tailor-made adoptive immunotherapy provided by the invention. A transplanted HA-1 positive recipient, transplanted with HA1 negative (or for that matter HA-1 positive) bone marrow and suffering from recurrent disease (relapse), i.e. HA-1 positive tumor cells, can be treated with (again) an agent (as above) which specifically recognizes a peptide according to the invention (a HA-1 peptide) as presented on hematopoietic cells, which agent induces elimination of the cells presenting said peptide. In case of HA-1 positive bone marrow being transplanted to the HA-1 positive recipient, it is still essential (in case of recurrent disease) to eliminate all HA-1 positive cells even though this includes the transplanted material, because otherwise the HA-1' positive tumor will kill the recipient. To avoid the latter case the patient can be re-transplanted, if necessary. In such therapy protocols it is possible to first employ adoptive immunotherapy with agents (cells, antibodies, etc.) which specifically recognize and eliminate specific peptide expressing cells (e.g tumor cells) that need to be destroyed, after which in a second phase the patient is reconstituted with BMT cells replacing the killed cells. The invention thus provides additional (or even substituting) protocols to other therapeutic measures such as radiation.

A CTL capable of specifically killing a cell presenting HA-1 in the context of a suitable HLA class I molecule is said to be an HA-1 specific CTL, even in cases wherein said CTL was raised (educated) against a different peptide. A (poly)peptide is said to comprise an HA-1 antigen when a suitable part of said (poly)peptide is recognized by the afore mentioned HA-1 specific CTL.when said part is presented in the context of a suitable HLA molecule. Now that the invention discloses that non-hematopoietic tumor cells express HA-1 it is possible to use this information for instance in developing diagnostic tools. Considering that normal non-hematopoietic cells do not express HA-1, it is possible to discriminate between a tumor non-hematopoietic cell and a normal non-hematopoietic cell, on the basis of HA-1 gene expression. This can be done on the protein (peptide) level and/or on the nucleic acid. The invention therefore further provides a method for marking a non-hematopoietic tumor cell comprising incubating said cell with a molecule capable of specifically binding to an HA-1 mHag presented in the context of HLA class I, or capable of specifically binding to a nucleic acid encoding said HA-1 mHag. Means and methods for determining the presence of HA-1 polypeptide or mRNA in a cell are well known to the person skilled in the art. Examples of detection methods are described in WO 99/05313 which is herein incorporated by reference. These methods may be combined with other detection and/or cell type characterization methods (for instance for expression products of other genes or microscopy) to exclude the presence of HA-1 expressing hemopoietic cells. The cell comprising said molecule capable of specifically binding to an HA-1 mHag presented in the context of HLA class I, or capable of specifically binding to a nucleic acid encoding said HA-1 mHag are also part of the invention.

### Examples

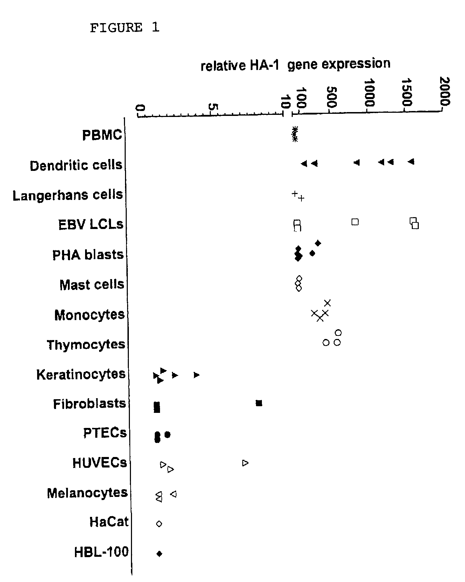

To confirm the hematopoietic system restricted tissue distribution, earlier analyzed by HA-1 specific CTLs, HA-1 mRNA levels were analyzed by quantitative real-time PCR (13) in eight different hematopoietic and six different non-hematopoietic cell types. Only cells of hematopoietic origin expressed significant levels of the HA-1 gene (Fig.1). No significant HA-1 gene expression was detected in cells of non-hematopoietic origin: i.e. keratinocytes, dermal fibroblasts, proximal tubular epithelial cells (PTECs), human umbilical vein endothelial cells (HUVECs), melanocytes and SV 40 immortalized breast cell lines HaCaT and HBL 100 (Fig.1).

Next, we investigated the HA-1 gene transcription levels in thirty -five epithelial tumor cell lines derived from different carcinomas (Table1). The HA-1 gene transcription, analyzed by quantitative real time RT-PCR, revealed significant HA-1 mRNA in twenty-six out of the thirty-five cell lines of various malignant origins. Table 1 also lists the results of the common leukocyte antigen CD45. We compared the HA-1 and CD45 RNA expression in various hematopoietic cells. Both genes are expressed in hematopoietic cells to comparable levels (data not shown). None of the tumor cell lines showed significant CD45 gene expression. This shows that HA-1 transcription observed in the tumor cell lines is specific and not due to contaminating HA-1 positive hematopoietic cells (Table 1).

Functional recognition by HA-1 specific CTLs is a prerequisite for tumor specific targeting in immunotherapeutical settings. The mHag HA-1 locus encodes two alleles i.e. the HA-1H and the HA-1R allele. The HA-1^{H} allele is the T cell epitope that is recognized by the HLA-A2 restricted CTL (14).

Therefore, we executed CTL recognition studies (15) on the tumor cell lines that expressed both the HLA-A2 restriction molecule and the HA-1H T cell epitope required for the HLA-A2 restricted HA-1 specific CTL recognition . Hereto, all tumor cell lines listed in table 1 were HLA and HA-1 genotyped (14). Table 2 shows significant HA-1 CTL lysis on four of the five cell lines by two HA-1 specific clones which could be enhanced in all cases by IFNγand TNFα treatment of the target cells. The colon carcinoma cell line CaCo-2 was only recognized by one of the two HA-1 CTL clones.

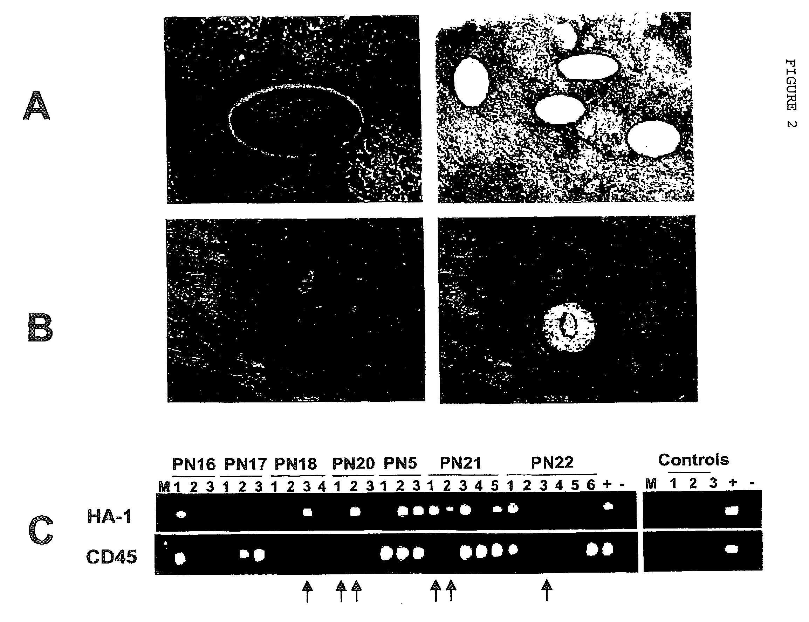

With the demonstrated functional expression of HA-1 by epithelial tumor cell lines, we expected that HA-1 is also expressed by epithelial tumors in vivo. However, given the expression of HA-1 by cells of the hematopoietic lineage and in view of the virtual omnipresence of hematopoetic cells in tumors, spurious positive results of a PCR analysis caused by contaminating hematopoietic cells should be avoided. To this end, we applied laser-mediated micro-dissection to cryosections of fresh frozen cancer samples without no microscopically visible leukocyte infiltration (16, Fig.2A). As control, we used micro-dissected normal breast glands from three patients that underwent breast reduction surgery (Fig 2B). By the applied micro-dissection method the selected area is cut by a laser beam and directly catapulted into the reaction tube, practically excluding contamination by surrounding tissue. Of twelve tumors obtained from patients with breast and lung cancers and the three biopsies from normal breast tissue, areas of 10,000-60,000 µm² in total (comprising about 30 - 200 cells) were laser-micro-dissected. mRNA was isolated, reverse transcribed and amplified with a recently developed global amplification method (17). Successful global amplification of cDNA was checked by established gene specific amplification of the two housekeeping genes b-actin and EF-1a and cDNA array hybridization (not shown). Following dilution of the primary PCR products, specific primers served to detect HA-1 gene expression (18). While seven of twelve tumors were positive for HA-1, all normal breast glands were negative (Fig.2C). The identity of the PCR bands as HA-1 was confirmed by Southern blotting (not shown). We used CD45 gene specific PCR to test whether HA-1 expression might be attributed to single infiltrating leukocytes or intravascular cells that had escaped our attention. Absence of CD45 mRNA would provide strong evidence that the HA-1 signal originates from the epithelial tumor cells in vivo. Indeed, four of seven tumor samples solely expressed HA-1 (Fig.2C, arrows) in at least one of the micro-dissected areas, whereas three tumors co-expressed CD45 and HA-1 prohibiting evaluation of their HA-1 status. Therefore, HA-1 was found to be expressed in at least 30% human primary tumors of epithelial origin in vivo.

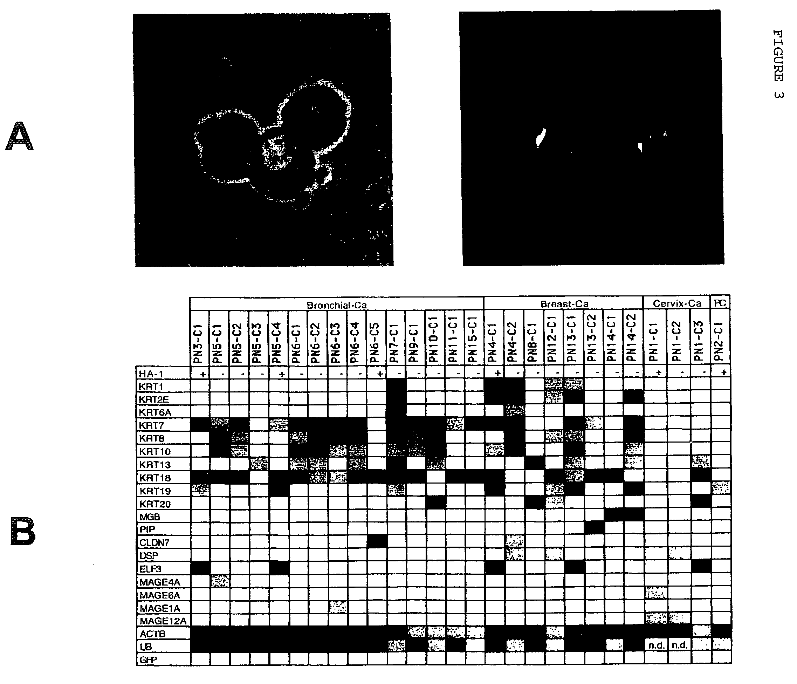

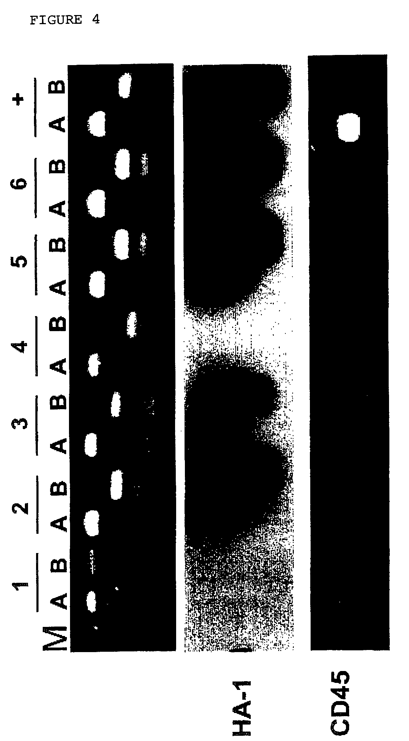

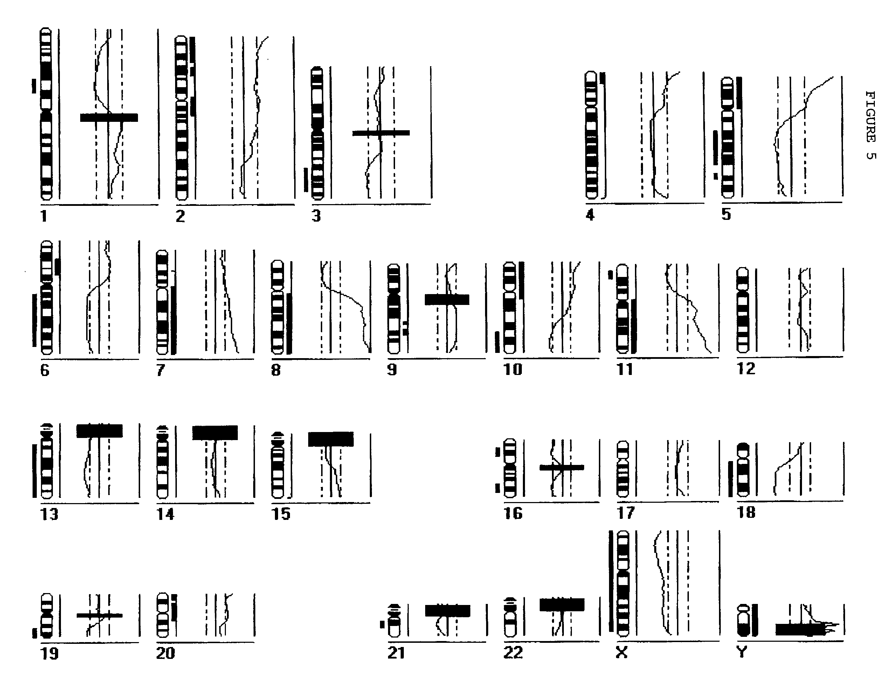

Since contamination by CD45 positive non epithelial cells could not be absolutely excluded as cause of the encountered CD45 expression in some of the micro- dissected tumor areas, we resorted to HA-1 analysis of single tumor cells or defined cell clusters freshly isolated from bone marrow or lymph nodes of cancer patients (Fig.3A). Single disseminated cancer cells were detected in cell suspensions prepared from bone marrow and lymph node samples with a fluorescent labeled monoclonal antibody against the epithelial cell adhesion molecule (EpCAM) as marker (19). In total, twenty-seven single tumor cells or small cell clusters were isolated by micromanipulation from fifteen cancer patients (Fig.3A). For cDNA analysis, the same global amplification technique was applied that was used for the micro-dissected tumor areas, enabling faithful detection of expressed transcripts in single cells (17). The labeled cDNAs were hybridized to an array including specific epithelial marker genes such as the cytokeratin family members (KRT), mammaglobin (MBG) and prolactin induced protein (PIP) as markers for breast-derived cells, and the transcription factor ELF3. Further evidence of epithelial origin was provided by claudin 7 (CLDN7) and desmoplakin I (DSP) both involved in epithelial cell adhesion. As indicator of malignancy, the expression of MAGE genes was analyzed, the transcripts are found in spermatogonal cells and exclusively in various cancer cells, hence the designation cancer-testis genes. In addition, we evaluated the cells for markers of hematopoietic cells such as the T cell receptor, CD45, CD33, CD34, CD37, CD38, and CD16. The isolated cells expressed none of the hematopoietic markers (not shown). Expression of cytokeratins and other epithelial markers indicated their epithelial origin (Fig.3B). In some cases the cells were positive for one or more MAGE genes suggesting their tumor origin, despite down-regulation of cytokeratin mRNA (Fig.3B). All cells were then tested for HA-1 and CD45 expression by gene specific PCR; the HA-1 amplification products were subsequently confirmed by restriction enzyme digest and by Southern blotting (18). Six of the twenty-seven cells expressed the HA-1 gene and none of them expressed the CD45 gene (Fig.4). The HA-1 significant transcripts were observed in samples derived from breast cancer (PN4-C1), bronchial carcinoma (PN3-C1, PN5-C1, PN6-C5), prostate cancer (PN2-C1) and cervical cancer (PN1-C1). From two of the HA-1 positive cells (PN5-C4, PN3-C1) we could besides mRNA also evaluate their DNA by a recently described method (C.A. Klein submitted and 19). The isolated DNA was subjected to whole genome amplification and comparative genomic hybridization (CGH). Both cells harbored multiple genomic alterations, lending ultimate proof of their malignant nature (Fig.5).

### References..

1. A. Butturini and R. P. Gale, Bone Marrow Transplant. 3, 185 (1988).
2. M. M. Horowitz et al., Blood 75, 555 (1990).
3. R. Childs et al., N.Engl.J.Med. 343, 750 (2000).
4. B. Eibl et al., Blood 88, 1501 (1996).
5. R. Ben Yosef, R. Or, A. Nagler, S. Slavin, Lancet 348, 1242 (1996).
6. A. N. Houghton, M. L. Meyers, P. B. Chapman, Surg.Clin.North Am. 76, 1343 (1996).
7. J. O. Bay et al., Bone Marrow Transplant 25, 681 (2000).
8. E. Goulmy, Immunol.Rev. 157, 125 (1997).
9. E. Goulmy, Curr.Opin.Immunol. 8, 75 (1996).
10. M. M. de Bueger, A. Bakker, J. J. van Rood, W. F. van der Woude, E. Goulmy, J.Immunol. 149, 1788 (1992).
11. D. van der Harst et al., Blood 83, 1060 (1994).
12. J. H. F. Falkenburg et al., J.Exp.Med. 174, 27 (1991).
13. Total RNA was prepared from subconfluent layers of the adherent cell cultures using the RNAzol method (Cinaa/ Biotecx Laboratories, Houston, TX) according to the manufacturer's description. cDNA was synthesized using 2 mg RNA and random hexameric primers. PCR amplification and quantification were performed using the Taqman PCR assay (PE Applied Biosystems 7700 Sequence Detector, Foster City, CA). We used comparative quantification normalizing the HA-1 and CD45 gene to an internal standard gene, the ubiquitously expressed housekeeping gene porphobilinogen deaminase (PBGD). To allow calculation of relative levels of expression, we used the KG-1 cell line, which expresses both genes, as a standard. The HA-1 and CD45 expression levels of the test samples were calculated as percentages of HA-1 and CD45 expression levels in the reference cell line KG-1. All samples tested which showed expression levels below 10% in the real time quantitative PCR did not produce detectable PCR fragments in a standard PCR. Therefore, expression levels < 10% are considered as not significant. The relative quantification was calculated by the linear calibration function between the threshold cycle (Ct) value and the logarithm of the initial starting quantity (N) were Ct=-3.31 log (N)+26.1, Ct=-3.5log(N)+21.6 and Ct=-3.41log(N)+25.6 for HA-1, CD45 and PBGD, respectively. The HA-1, CD45 and PBGD expression were quantified in all test samples by using these calibration functions.
14. J. M. M. den Haan et al., Science 279, 1054 (1998).
15. Tumor cell lines were used as target cells in a 4hr 51Cr release assay. The tumor cells from subconfluent cultures were harvested and dispensed at 2500 cells/ well in 96 wells flat bottomed microtiter plates and allowed to attach either in the presence or the absence of rINFg (250U/ml, Gentech, San Francisco, CA) and TNFa (250U/ml, San Francisco, CA) for 48 hrs. The tumor cells were labeled with 51Cr for 1hr and the experiments were performed in sixplicates. The percentage specific lysis was calculated as follows: % specific lysis = (experimental release- spontaneous release)/ ( maximal release-spontaneous release) X 100.
16. Preparation of cryosections. Sections (5µm) from freshly shock frozen primary tumors were placed on a polyethylene membrane on a glass slide, stained with Meyer's hematoxylin and dehydrated in 70%, 90% and 100% ethanol. The PALM Microbeam system (Bernried, Germany) was used for microdissection and catapulting.
17. Detection of disseminated cells, global amplification of micro- dissected areas and of single cells from bone marrow and lymph nodes was performed as described in detail (Klein et al., submitted). Briefly, the viable bone marrow or lymph node samples were stained for 10 min. with 10 µg/ml monoclonal antibody 3B10-C9 in the presence of 5% AB-serum. 3B10-C9-positive cells were detected with B-phycoerythrin-conjugated goat antibody to mouse IgG (The Jackson Laboratory) and transferred to PCR-tubes on ice. Oligo-dT beads in 10µl lysis buffer (Dynal) were added, the cells lysed, tubes rotated for 30 min. to capture mRNA. 10 µl cDNA wash buffer-1 (50 mM Tris-HCl, pH 8,3, 75mM KCl, 3mM MgCl2, 10mM DTT, supplemented with 0.5% Igepal (Sigma)) was added and mRNA bound to the beads washed in 20 µl cDNA wash buffer-2 (50 mM Tris-HCl, pH 8,3, 75mM KCl, 3mM MgCl2, 10mM DTT, supplemented with 0.5% Tween-20 (Sigma)), transferred to a fresh tube and washed again in cDNA wash buffer-1. mRNA was reverse transcribed with Superscript II Reverse Transcriptase (Gibco BRL) using the buffers supplied by the manufacturer supplemented with 500 µM dNTP, 0.25% Igepal, 30 µM CFL5c8 primer (5'-(CCC)5 GTC TAG ANN (N)8-3') and 15 µM CFL5cT (5'-(CCC)5 GTC TAG ATT (TTT)4 TVN, at 44°C for 45 min. Samples were rotated during the reaction to avoid sedimentation of the beads. cDNA remained linked to the paramagnetic beads via the mRNA and was washed once in the tailing wash buffer (50 mM KH2PO4 pH 7.0, 1mM DTT, 0.25% Igepal). Beads were resuspended in tailing buffer (10 mM KH2PO4, pH 7.0, 4 mM MgCl2, 0.1 mM DTT, 200 µM GTP) and cDNA-mRNA hybrids were denatured at 94 °C for 4 min, chilled on ice, 10 U TdT (MBI-Fermentas) added and incubated at 37°C for 30 - 60 min. After inactivation of the tailing enzyme (70°C, 5 min), PCR-Mix I was added consisting of 4 µl of buffer 1 (Roche, Taq long template), 3% deionized formamide (Sigma) in a volume of 35 µl. The probes were heated at 78°C in the PCR cycler (Perkin Elmer 2400), PCR Mix II, containing dNTPs at a final concentration of 350 µM, CP2 primer (5'-TCA-GAA-TTC-ATG-CCC-CCC-CCC-CCC-CCC-3', final concentration 1.2 µM) and 5 Units of the DNA Poly-Mix was added, (Roche, Taq Long Template) in a volume of 5 µl for a hot start procedure. Forty cycles were run at 94°C for 15 sec, at 65°C, 30°C, 68°C for 2 min. for the first 20 cycles and a 10 sec- elongation of the extension time each cycle for the remaining 20 cycles, and a final extension step at 68°C, 7 min
For expression profiling digoxigenin-UTP was incorporated by PCR using 0.1-1µl of the original PCR amplified cDNA fragments reamplification in the presence of 50 µM dig-dUTP (Roche), 300 µM dTTP, and other dNTPs at a final concentration of 350 µM. Reamplification conditions were essentially as described above, modifications were the use of 2.5 Units of the DNA Poly Mix. Initial denaturation at 94°C for 2 min. followed by 12 cycles at 94°C, 15 sec, 68°C, 3 min. and a final extension time of 7 min. Filters were prehybridised overnight in the presence of 50 mg/ ml E.coli and 50 mg/ ml pBS DNA in 6 ml Dig-easy Hyb buffer (Roche). Labeled PCR products from single cells were added in a concentration of 1.5 µg / ml mixed with 100 µg herring sperm to prehybridization buffer, and hybridized for 36-48 hours. Stringency washes were performed according to the RocheTM digoxigenin hybridization protocol adding two final stringency washes in 0.1x SSC +0.1 % SDS for 15 min at 68°C. Detection of filter bound probes was performed according to the Digoxigenin detection system protocol supplied with the kit (Roche).
18. Amplification of HA-1 and CD45. All samples were analyzed by two primer pairs for HA-1: HA-1 (I) (forward: 5'-GAC GTC GTC GAG GAC ATC TCC CAT-3'; reverse: 5'-GAA GGC CAC AGC AAT CGT CTC CAG-3') and HA- 1 (II) (forward: 5'-ACA CTG CTG TCG TGT GAA GTC-3'; reverse: 5'-TCA GGC CCT GCT GTA CTG CA-3'). CD45 forward: 5'- CTG AAG GAG ACC ATT GGT GA) and reverse 5'-GGT ACT GGT ACA CAG TTC GA-3' primer. Amplification products of the HA-1 (I) primers were digested with the restriction enzyme BstU I and amplification products of the HA-1 (II) primers with Hinf I. Southern blot was performed according to standard protocols.
19. C. A. Klein et al., Proc.Natl.Acad.Sci.U.S.A 96, 4494 (1999).
20. H. J. Kolb et al., Blood 76, 2462(1990).
21. S. Slavin et al., Blood 91, 756 (1998).
22. T. Mutis et al., Blood 93, 2336 (1999).
23. N. T. Ueno et al., J.Clin.Oncol. 16, 986 (1998).
24. S. Braun et al., N.Engl.J.Med. 342, 525 (2000).
25. C. A. van Els et al., Immunogenetics 35, 161 (1992).
26. The following cell lines were kindly provided by: MDA-MB 231, 734 B, MCF-7, ZR75-1 by Dr. B. Eibl (Dept. of Clinical Immunobiology, University Hospital of Internal Medicine, Innsbruck, Austria); HBL-100 cell line, Mel 93.04C and LB 33 by Dr. S. Osanto (Dept. of Oncology, Leiden University Medical Center, Leiden, The Netherlands); BT-20, BT, MEWO, E9, BT, MNT and BA by Prof. G.C. de Gast (UMC, Utrecht, The Netherlands); GLC2, GLC 8, and GLC 36 by Prof. L. de Leij (Dept. of Clinical Immunology, AZG, Groningen, The Netherlands); BB74/ 2940, KUL 68/ 3636 and BB 49/ 1413 by Dr. F. Brasseur, (Ludwig Institute for Cancer Research, Brussels, Belgium); HuH7 and HepG2 by Dr. B.J. Scholte (Erasmus University Rotterdam, The Netherlands); HT-29 (ATCC: HTB-38) and Caco-2 (ATCC: HTB-37) are ATCC cell lines.

**Table 1:**

| HA-1 and CD45 gene expression in tumor cell lines. Percentages represent HA-1 and CD45 gene expression relative to the standard cell line KG-1 as analyzed by quantitative real time PCR. | | | |
|---|---|---|---|
| **Tumor type** | **Cell line** | **% CD45** | **% HA-1** |
| Breast cancer | ZR75-1 | ≤10 | 54 |
| | BT-20 | ≤10 | 40 |
| | 734B | ≤10 | 27 |
| | T47 D | ≤10 | 17 |
| | MDA-MB231 | ≤10 | 15 |
| | MCF-7 | ≤10 | ≤10 |
| | BT 474 | ≤10 | ≤10 |
| | | | |
| Melanoma | Mel 93.04 | ≤10 | 68 |
| | KUL 68/3636 | ≤10 | 67 |
| | BB 74/2940 | ≤10 | 57 |
| | MNT | ≤10 | 27 |
| | LB33 | ≤10 | 24 |
| | BT | ≤10 | 15 |
| | 453 Ao | ≤10 | 12 |
| | 518A | ≤10 | ≤10 |
| | E9 | ≤10 | ≤10 |
| | MEWO | ≤10 | ≤10 |
| | | | |
| Lung carcinoma | GLC 36 | ≤10 | 22 |
| | GLC 8 | ≤10 | ≤10 |
| | GLC 2 | ≤10 | ≤10 |
| | | | |
| Renal Cell Carcinoma | MZ 1851 | ≤10 | 29 |
| | MZ 1752 | ≤10 | 13 |
| | MZ 1774 | ≤10 | ≤10 |
| | BA | ≤10 | ≤10 |
| | | | |
| Hepatoma | HuH7 | ≤10 | 37 |
| | HepG2 | ≤10 | 35 |
| | | | |
| Colon carcinoma | SW 707 | ≤10 | 147 |
| | CaCo-2 | ≤10 | 81 |
| | SW 480 | ≤10 | 70 |
| | SW 2219 | ≤10 | 48 |
| | SW 620 | ≤10 | 28 |
| | Col 205 | ≤10 | 21 |
| | SW 948 | ≤10 | 12 |
| | HT29 | ≤10 | 11 |
| | | | |
| Head and Neck cancer | BB 49/1413 | ≤10 | 54 |

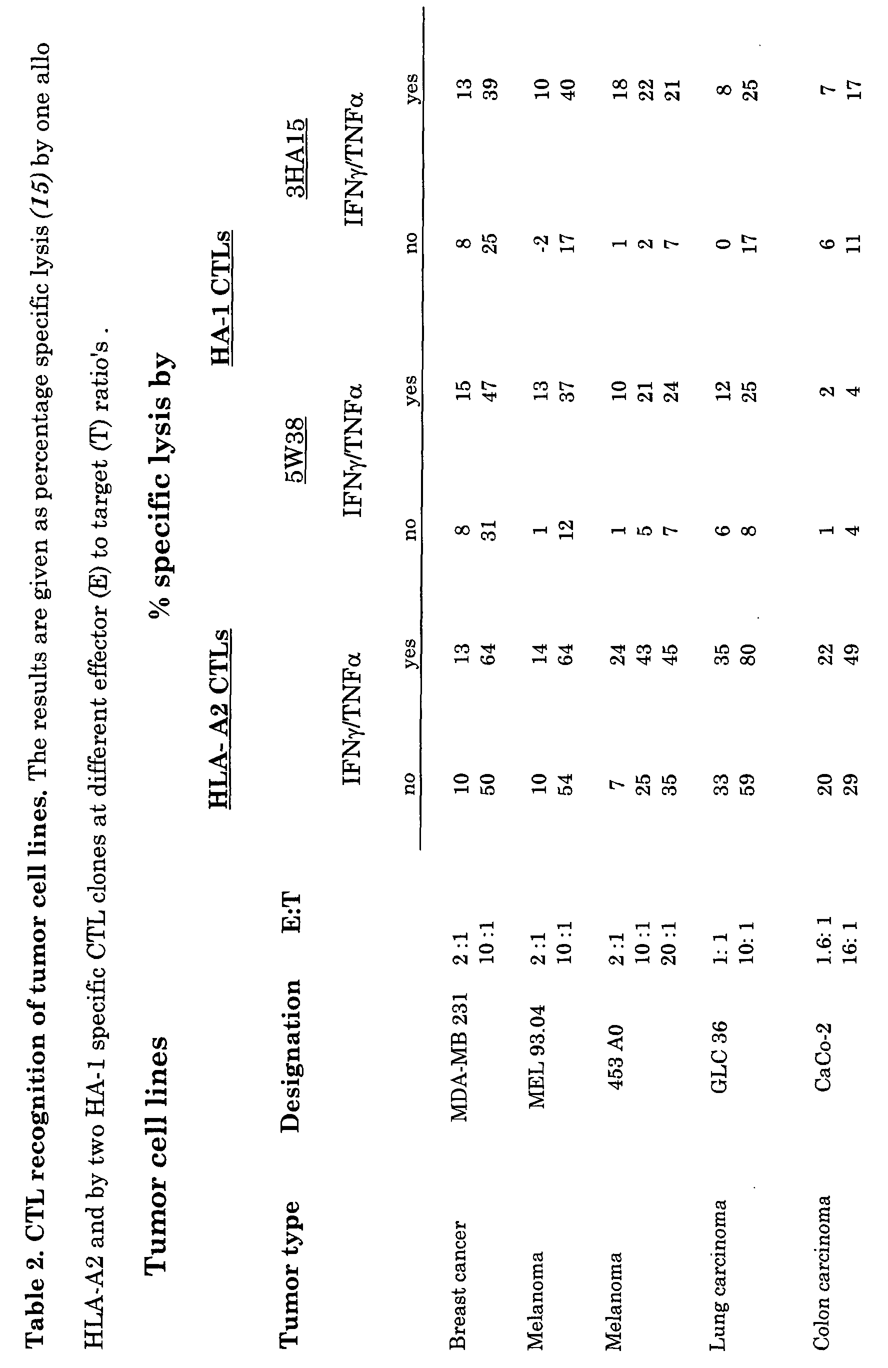

### Brief description of the figures

**Figure 1:** HA-1 gene expression in hematopoietic and non-hematopoietic cells. The relative HA-1 gene expression levels were determined by a calibration function generated from RNA of the HA-1 positive KG-1 cell line. Cells of hematopoietic origin tested were: *PBMCs (n=3), Dendritic cells (n=6), + Langerhans cells (n=2), £ EBV-LCLs (n=5), uPHA blasts (n=6), Mast cell lines (n=3), I Monocytes (n=4), □ Thymocytes (n=3). Cells of non-hematopoietic origin tested were: □ Keratinocytes (n= 5), ¢ Fibroblasts (n=2), ―PTECs (n=3), r HUVECs (n=3), s Melanocytes (n=3), two SV40 immortalized breast cell lines: HaCat and ¿ HBL-100.
**Figure 2:** HA-1 and CD45 expression of micro-dissected tissue samples **A and B**. Micro-dissection of primary tumor (adenocarcinoma of the lung) and normal breast gland, respectively.
   **C.** Three to six areas from a tumor samples (about 10,000 - 50,000 µm2 of a 5 µm section) were individually analyzed by gene specific PCR for HA-1 (primer HA-1 (II)) and CD45. The same was done using pooled cDNA from several milk ducts (in total 60,000 µm2 each) from normal breast tissue of three different donors (controls 1-3). Lane numbering indicates the different micro-dissected areas, PN = patient number. Arrows point to tumor areas without contaminating hematopoietic cells but positive HA-1 signal.
**Figure 3**: Isolation and gene expression analysis of single disseminated cancer cells or small tumor cell clusters.
   **A**. Three-cell cluster (PN5-C4) after micromanipulator-assisted isolation from a cell suspension of a lymph node preparation. All cells of the cluster are intensively stained by the EpCAM antibody.
   **B.** Gene expression profiling on cDNA array of isolated tumor cells. HA-1 expression after standard RT-PCR is given in the first line. The gray shades represent the signal intensity (from light gray = weak signal to black = strong signal).
**Figure 4:** HA-1 expression of disseminated cancer cells Cells positive for the HA-1 (II) primer pair were digested with Hinf I, blotted and hybridized with the respective probe. M= size marker, A = undigested PCR product, B = Hinf I digested product; lane 1 = PN12-C1; lane 2 = PN4-C1; lane 3 = PN3-C1; lane 4 = PN5-C1; lane 5 = PN6-C5; lane 6 = PN2-C1; + = HT29 for HA-1 and normal bone marrow for CD45.
**Figure 5**. CGH profile of cell PN3-C1. Each chromosome is represented by its ideogram and numbered. Deletions are marked with a red bar (e.g. loss of chr.13) at the left and gains with a green bar (e.g. gain of chr. 8q) at the right side of the chromosome symbol.

## Claims

1. A method for eliminating an tumor cell presenting a HA-1 minor histocompatibility antigen (mHag) in the context of HLA class I, wherein said elimination is induced directly or indirectly by specific recognition of said mHag in said context, **characterized in that** said tumor cells comprise non-hematopoietic cells.

2. A method for killing a human cell functionally expressing an HA-1 mHag in the context of HLA class I, comprising incubating said cell with a cytotoxic T lymphocyte (CTL) specific for said mHag presented in said context or incubating said cell with a functional equivalent of said CTL, the method **characterized in that** said human cell comprises a non-hematopoietic tumor cell.

3. A method for determining whether a cell expresses functional levels of an HA-1 mHag in the context of HLA class I, comprising incubating said cell with a cytotoxic T lymphocyte (CTL) specific for said HA-1 mHag presented in said context and determining whether said cell and/or said CTL is affected.

4. A method for marking a non-hematopoietic tumor cell comprising incubating said cell with a molecule capable of specifically binding to an HA-1 mHag presented in the context of HLA class I, or capable of specifically binding to a nucleic acid encoding said HA-1 mHag.

5. A non hematopoietic tumor cell comprising a molecule capable of specifically binding to an HA-1 mHag presented in the context of HLA class I, or capable of specifically binding to a nucleic acid encoding said HA-1 mHag.

6. A method for at least in part inhibiting expansion of an tumor cell in an individual comprising providing said individual with a CTL specific for an HA-1 mHag presented in the context of HLA class I, or a functional equivalent of said CTL, the method **characterized in that** said tumor cell comprises a non-hematopoietic tumor cell presenting said HA-1 mHag in the context of said HLA class I

7. A method according to claim 6, wherein said individual is provided with said CTL by providing said individual with a graft comprising hematopoietic cells of a donor.

8. A method according to claim 7, wherein said individual is provided with said CTL as a result of the induction of a Graft versus Tumor reaction in said individual.

9. A method according to any one of claims 6-8, wherein said indidvual is vaccinated with a vaccine comprising an HA-1 antigen or a functional equivalent thereof.

10. A method for generating a CTL capable of binding to an HA-1 mHag presented in the context of HLA class I comprising the step of administering to an individual that comprises a mismatch for said HA-1 mHag, a non-hematopoietic tumor cell expressing said HA-1 mHag presented in said context.

11. A CTL capable of binding to an HA-1 mHag presented in the context of HLA class I obtained by a method according to any one of claims 610.

12. Use of an antigen specific T cell comprising a specificity for HA-1 presented in the context of MHC class-I for the preparation of a medicament for the treatment of disease related at least in part to non-hematopoietic tumor cells.

13. Use of a molecule capable of specifically binding an HA-1 mHag in the context of HLA class I, for the preparation of a medicament for the treatment of disease related at least in part to non-hematopoietic tumor cells.

14. Use of a HA-1 antigen or a functional equivalent thereof for the preparation of a vaccine for the treatment of cancer that is caused by non-hematopoietic tumor cells.

15. Use of HA-1 antigen for inducing and/or enhancing the generation of HA-1 specific cytotoxic lymphocytes in an HA-1 negative donor of lymphocytes, wherein said generated lymphocytes are used for the preparation of a medicament for the treatment of cancer that is caused by non-hematopoietic tumor cells.
